(19) Europäisches Patentamt
European Patent Office
Office européen des brevets

(11) Publication number: **0 184 909 A2**

(12) # EUROPEAN PATENT APPLICATION

(21) Application number: 85308277.4

(22) Date of filing: 13.11.85

(51) Int. Cl.⁴: **C 12 Q 1/00, C 12 M 1/40**

(30) Priority: **13.11.84 GB 8428599**

(43) Date of publication of application: **18.06.86 Bulletin 86/25**

(84) Designated Contracting States: **AT BE CH DE FR GB IT LI LU NL SE**

(71) Applicant: **Genetics International, Inc., 222 Third Street Suite 0174, Cambridge Massachusetts 02142 (US)**

(72) Inventor: **Albery, Wyndham John, Flat 8, 53 Queens Gardens Paddington, London (GB)**
Inventor: **Bartlett, Nigel Philip, 10 New Street, Kenilworth Warwickshire (GB)**
Inventor: **Craston, Derek Harry, "Riverdell" St. Johns Road, Crowborough East Sussex (GB)**
Inventor: **Bycroft, Mark, 14 The Cloisters Salthouse Lane, Beeston Nottingham (GB)**
Inventor: **Jones, Christopher Peter, 61 Portsmouth Road, Woolston Southampton (GB)**

(74) Representative: **Clifford, Frederick Alan et al, MARKS & CLERK 57/60 Lincoln's Inn Fields, London WC2A 3LS (GB)**

(54) **Bioelectrochemical assay and apparatus.**

(57) Salts of TCNQ (7,7,8,8-tetracyano-p-quinodimethane such as the copper (dipyridylamine), tetrathiafulvalene (TTF), ferrocene, triethylamine and quinoline salts are used in the fabrication of electrodes for direct electrical determination of the progress of flavoprotein enzyme reactions, the enzyme preferably being associated at the electrode surface. Choline oxidase and pyruvate oxidase for phosphate assay, are preferred enzymes and the salt $TTF^+TCNQ^-$ is the preferred salt. $TTF^+TCNQ^-$ also permits direct electrical coupling NAD/NADH to the electrode.

Tetracyanoquinodimethane (Acceptor) 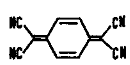 (a)

Copper di-pyridylamine 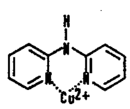 (b)

Tetrathiafulvalene 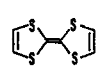 (c)

Ferrocene 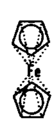 (d)

Triethylammonium 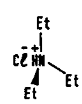 (e)

Quinoline 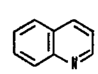 (f)

M&C FOLIO 230P48776 WANGDOC.0227s

BIOELECTROCHEMICAL ASSAY AND APPARATUS

The present invention is concerned with a bioelectrochemical assay and an apparatus for performing the said assay.

Our European Patent Application 82305597 describes and claims a sensor electrode composed of electrically conductive material and comprising at least at an external surface thereof the combination of an enzyme and a mediator compound which transfers electrons to the electrode when the enzyme is catalytically active.

The purpose of such an electrode is to detect the presence of, measure the amount of and/or monitor the level of one or more selected components capable of undertaking a reaction catalysed by the said enzyme.

Examples of electrode configurations, mediators and uses are given in that patent application.

Many dehydrogenase enzymes use the co-enzyme Beta-nicotinamide adenine dinucleotide ($NAD^+$) producing the reduced form NADH. The rate or amount of NADH production is used for a wide range of assay

systems to assay either the enzyme concentration or the enzyme activity, or (where the concentration of the enzyme is known) the substrate concentration. Such measurements are presently performed with a bench-top spectrophotometer, making use of the 340nm absorbtion by the reduced form NADH.

It is known that the in-situ regeneration of $NAD^+$ by oxidation of NADH can be performed electrochemically, and it has been postulated that this could be the basis of an electrochemical sensor. Unfortunately, the direct oxidation of NADH at a metal electrode proceeds through radical intermediates and requires the use of overvoltages large enough to invite interference from other oxidisable substances present in the reaction media. One approach to overcoming this problem has been to employ mediator substances. However the electrode configurations which have previously been offered have had an inherent chemical instabillity, and have proved unsuccessful in certain applications.

A further group of enzymes, with which the present invention is concerned, are the flavoproteins i.e. enzymes which utilise flavin adenine dinuculeotide, riboflavin mononucleotide or derivatives thereof. In the course of the biochemical reactions in which these compounds are involved the isoalloxazine ring system of

the flavin coenzyme is reduced. These enzymes are found widely e.g. in the oxygen-linked dehydrogenation of substrates such as amino-acids, the cytochrome-linked dehydrogenation of the initial members of the particle bound respiratory chain (i.e. NADH and succinate in most cells; alpha-glycerophosphate, choline, sarcosine, fatty-acid CoA's in appropriate mitochondria, and D- and L-lactate in aerobically grown yeasts), and in the NAD(P)-linked dehydrogenation of low-potential substrates, such as reduced ferredoxin and other non-haem iron NHI carriers. Enzymes in the first group usually contain only a flavin prosthetic group, while those in the second group frequently contain additional components which are usually metals.

In both the above cases, there are few effective assay systems which are both non-spectrophotometric and inexpensive to perform, and which have sufficient resolution to detect the presence of the enzyme or to monitor its activity at physiologically significant levels.

The present invention is concerned with novel electrode materials and configurations, the manufacture thereof, the use thereof in a preferred mode, and the assay techniques available using such electrodes.The early work in this field concerned firstly the use of glucose

oxidase in combination with the conductive charge-transfer complex composed of either N-methylacridinium (NMA) or N-methylphenazinium (NMP) as cation, together with the anion radical tetracyano-p-quinodimethane (TCNQ); and secondly, the use of cytochrome $b_2$ and horse-radish peroxidase adsorbtion followed by hydrogen peroxide reduction and L-lactate oxidation.

At the time of this early work it was considered that the NMP and the NMA were the active groups in the complex.

According to one aspect of the present invention there is provided an electrode for use in an assay system, wherein the said electrode is at least in part made from a material(X) having one-dimensional electrical conduction properties, and wherein the material (X) is linked to the other components of the assay system via a $NAD^+$/NADH couple.

According to a further aspect of the present invention there is provided an electrode for use in assay system which electrode exhibits at an operative surface (a) a material having one-dimensional electrical conduction properties and (b) a flavoprotein enzyme chosen from glucose oxidase, L-aminoacid oxidase, D-aminoacid

oxidase, choline oxidase, xanthine oxidase, monoamine oxidase and pyruvate oxidase.

The invention, in a related aspect further provides an electrode for use in an assay system which electrode exhibits at an operative surface (a) a material having one-dimensional electrical conduction properties chosen from copper (dipyridylamine), tetrathiafulvalene, ferrocene, triethylamine and quinoline salts of 7,7,8,8-tetracyano p-quinodimethane, (TCNQ) together with (b) an enzyme located at least at the said operative surface of said electrode whereby charge is transferred to said electrode when the enzyme is catalytically active.

In 1965 W. A. Little of Stanford University proposed the theory of organic superconductors (Scientific American Vol 212, No 2. pages 21-27), wherein superconductors could be fabricated from a chain of carbon atoms with loosely bound valence electrons capable of forming "Cooper Pairs". Cooper Pairs are loosely bound two-electron systems which can move through a solid without losing energy through scattering, as the energy required to scatter a Cooper pair is greater than the binding energy which holds the two-electron system together, and far greater than the energy available within the lattice. Although this particular mechanism

has not been proven in this case, it should be understood that the invention extends to materials having these properties.

Conveniently the material(X) is an organic conductor.

Preferably the material(X) is a derivative or salt of 7, 7, 8, 8 tetracyano p-quinodimethane.

One of the important requirements for an organic conductor was originally thought to be that the molecules of the solid had to have large planar molecules in which the valence electrons are found predominantly above and below the planar framework. One of the first organic molecules of this type to be synthesised was 7, 7, 8, 8-tetracyano-p-quinodimethane (TCNQ) which was found to a poor conductor of electricity.

More preferably the salt of TCNQ is the Cu(di-pyridylamine), tetrathiafulvalene, ferrocene, triethylammonium or quinoline salt.

In a preferred embodiment of the invention, the material(X) comprises a tetrathiafulvalene (TTF) salt of 7, 7, 8, 8-tetracyano p-quinodimethane
The salt TTFTCNQ is particularly stable, and is more

stable than the other salts specifically exemplified herein. A particular utility of this compound is that it can be used in combination with a number of flavoprotein oxidases.

In one particular embodiment of the present invention the TTFTCNQ salt is used in combination with the a flavoprotein as listed above, especially with choline oxidase and pyruvate oxidase.

In a further preferred embodiment of the invention, the material(X) comprises an n-methyl phenazinum (NMP) salt of 7, 7, 8, 8-tetracyano p-quinodimethane

NMPTCNQ was first prepared by Melby (Canadian Journal of Chemistry 1965, 43, 1448) and was found to have a conductivity comparable to that of copper. Studies of the enzyme electrochemisty (Kulys et al.Anal Chim Acta 1982 138 19 and 1980 117 115) of this material have shown that it may enter into biochemical redox reactions, However, no previous worker has shown that the material can be employed with an NADH-containing system.

We have determined that one particularly useful feature of the embodiments which employ NMPTCNQ is that the electrode potential may be swept outside of the region

of electrode stability to dissolve the outer layers of the electrode in a controlled fashion, and thereby present a fresh surface to the electrolyte.

Accordingly, a further aspect of the invention resides in a method for the regeneration of an electrode for use in an electrochemical assay system, in which the potential of the electrode is swept outside of that range within which the outer layers of the electrode are stable to regenerate the electrode.

The above procedure is not possible with electrodes which have been modified with a covalent monolayer, or with a polymer layer containing redox groups.

In the solid form of the mixture, the TCNQ and (for example) TTF molecules stack in separate, parallel columns and electrons are transferred from the TTF stack (donor) to the TCNQ stack (acceptor). Due to this electron transfer there can be a net motion of electrons along both stacks, hence the material is conductive.

This material was found to have the surprising property of anisotropic electrical conduction; that is, the material is highly conductive in one direction only, with the most favourable direction showing a five-hundred fold increase in conductivity over the

least favourable direction.

We have demonstrated the general applicability of TCNQ containing assay systems when employed with oxidases and dehydrogenases, either when these are NAD-linked or are flavoproteins with other prosthetic groups.

Various configurations of electrodes can be envisaged within the scope of the present invention. For example the following general types of electrode; where the material(X) is packed as a paste into the cavity of a cavity electrode; where the material(X) is drop coated onto a glassy carbon electrode, or where the material(X) is present as a single crystal.

In the most preferential embodiment of the invention the electrode further comprises an enzyme at least at an external surface thereof, whereby charge is transferred to the electrode when the enzyme is catalytically active. Preferably the enzyme is a flavoprotein, and is selected from the following group; Glucose Oxidase, Xanthine Oxidase, Choline Oxidase, L-amino acid Oxidase, D-amino acid Oxidase and Monoamine Oxidase.

All the materials studied show reactivity as electrodes for the reoxidation of glucose oxidase. However in most cases the background currents were large and tended to

drift. Thus one important feature in the choice of the TCNQ salt to be used as the electrode material is the background electrochemistry. For this reason TTF.TCNQ is the material of choice out of the five materials investigated.

A particularly useful and unexpected finding was that TTF.TCNQ could reoxidise choline oxidase, an enzyme for which no alternative electron acceptor to $O_2$ was previously known. It is envisaged that an acetylcholine sensor could be configured by the use of choline oxidase in conjuction with acetylcholine esterase. Furthermore an acetylcholine esterase sensor can be envisaged which has a supply of acetylcholine provided at the electrode surface together with choline oxidase, and in which choline produced by the action of any added acetylcholine esterase is assayed as described herein.

NMP.TCNQ also works well with the other flavoproteins, in addition to glucose oxidase, for example, Xanthine Oxidase and Monoamine Oxidase.

The invention will be further described by way of example and with reference to the accompanying figures wherein;

__Figure 1__: shows the structures of the various donors used, more particularly:

Figure 1a shows Tetracyanoquinodimethane (Acceptor),

Figure 1b shows Copper di-pyridylamine (Donor),

Figure 1c shows Tetrathiafulvalene (Donor),

Figure 1d shows Ferrocene (Donor),

Figure 1e shows Triethylammonium (as Chloride) (Donor),

Figure 1f shows Quinoline (Donor),

__Figure 2__: shows for comparison typical cyclic voltammograms A to E, as identified below for each of the electrode materials in background buffer,

__Figure 3__: shows a plot of the current (corrected for background) against concentration of glucose for a Quinolium/TCNQ packed cavity electrode when;

A: the electrode is covered with dialysis membrane contacted with a solution of Glucose Oxidase.

B: the electrode is dipped in Glucose oxidase for 1 hr and then washed before use.

C: the same procedure as B is followed after storage in buffer solution overnight,

__Figure 4:__ shows in longitudinal section a cavity electrode according to the present invention,

Figure 5; shows typical results for the reponse of a TTF.TCNQ electrode with adsorbed Glucose oxidase, as described in Example 9(b) below.

Figure 6; shows a plot of the corrected current as a function of the concentration of added glucose for a TTF.TCNQ electrode with adsorbed Glucose Oxidase and no membrane.

Figure 7; shows a current-vs-time plot for a TTFTCNQ/glucose oxidase electrode over 180 hours.

Figure 8; shows a plot of the response of a Choline Oxidase/TTFTCNQ electrode (Example 10a).

Figure 9; shows a plot of the response of a Xanthine Oxidase/TTFTCNQ electrode (Example 10b).

Figure 10; shows a further plot of the response of a Xanthine Oxidase/TTFTCNQ electrode, additions of xanthine being shown at "+".

Figure 11; shows a plot of the response of a L-amino acid Oxidase/TTFTCNQ electrode (Example 10c).

Figure 12; shows a plot of the response of a D-amino acid Oxidase/TTFTCNQ electrode (Example 10d).

Figure 13; shows a plot of the response of a Glucose Oxidase/TTFTCNQ electrode.

Figure 14 shows a plot of the response of a pyruvate oxidase/$TTF^+TCNQ^-$ electrode.

ELECTRODE MATERIALS

Samples of the following organic conductors were prepared by the methods given in the literature. L.R. Melby, R.J. Harder, W.R. Hertler, W. Mahler, R.E. Benson and W.E. Mochel, J.A.C.S. 84, 3374 (1962).

(A) Cu(di-pyridylamine) $TCNQ_2$

(B) Tetrathiafulvalene TCNQ (TTF.TCNQ)

(C) Ferricinium TCNQ

(D) Triethylammonium $TCNQ_2$

(E) Quinolinium $TCNQ_2$

In Figure 1 we give the structures of the various donors used. All compounds were obtained as shiny black microcrystalline solids.

PREPARATION OF ELECTRODES

Three methods for preparing samples of the organic conductors as electrode materials have been used for the

various compounds. It is also envisaged that the materials could be employed in the form of a pressed pellet. The three methods used are described below.

Example 1;Packed Cavity Electrode.

The microcrystaline compound was mixed with polyvinyl chloride in the ratio 9.1 : 1.4 by weight. The mixture was then made up into a thick slurry with a small quantity of purified tetrahydrofuran and the paste then packed firmly into the cavity of the cavity electrode. After smoothing of the front face of the cavity flush with the surface of the electrode the tetrahydrofuran was allowed to evaporate at room temperature and pressure for at least 30 minutes. Electrodes were washed with doubly distilled water (DDW) before use.

Example 2;Drop Coated Glassy Carbon.

The same mixture of the organic conductor and PVC as used in example 1 was dissolved in a slightly larger volume of purified tetrahydrofuran to make a solution which could be dropped onto the surface of the glassy carbon electrode from a dropping pipette. On evaporation of the solvent this left a film of material on the electrode surface. The quantity of material was controlled by varying the amount of solution dropped

onto the electrode. For thicker coatings the procedure was repeated two or more times. The electrodes were washed with DDW before use.

Example 3: Single Crystal Electrodes.

For those materials which gave sufficiently large single crystals, electrodes were made up from these. Contact was made to one end of the needle-shaped crystals using a fine copper wire and a small quantity of silver-loaded epoxy resin. The contacted crystals were then carefully fitted into the ends of glass capillaries and insulated using ordinary epoxy resin so that about one half of the crystal was exposed to the solution. The whole electrode assembly was left to cure overnight and washed with DDW before use.

ELECTROCHEMISTRY

All measurements were made in phosphate buffer pH 7.4 containing 150mM NaCl. All potentials are reported relative to the saturated calomel electrode (SCE).

Figure 2 shows for comparison typical cyclic voltammograms for each of the electrode materials in background buffer. Cyclic voltammograms for the various salts recorded in phosphate buffer pH 7.4 at 25°C and 10

mV/s.

Example 4: Use of Cu(dipyridylamine) $TCNQ_2$

Cu(dipyridylamine)$TCNQ_2$ was drop coated on glassy carbon (area 0.38 $cm^2$).

In the background buffer solution the capacitative currents in the cyclic voltammogram were found to increase dramatically with successive cycles between -200 and +600mV. The background currents observed at a fixed potential were very slow to stabilize and particularly sensitive to the choice of potential.

Using a drop coated glassy carbon electrode in the presence of glucose oxidase a response to glucose was observed but this was far from ideal due to the high background currents.

Example 5: Use of Tetrathiafulvalene TCNQ.

TTF.TCNQ was drop coated on glassy carbon (area 0.38 $cm^2$).

This material gave the best background electrochemistry with very low, stable background currents. It is the most promising of the compounds for detection of glucose

using glucose oxidase of those so far investigated, including NMP.TCNQ. This work is described in detail in Example 9.

Example 6: Use of Ferricinium TCNQ.

A ferricinium TCNQ single crystal was employed (area 2.0 $mm^2$).

The packed cavity electrode method gave very poor results for this material. This appeared to be the result of the formation of free ferrocene on dissolution of the compound in tetrahydrofuran. For this reason all experiments with this compound were conducted with single crystal electrodes.

In background buffer the accessible potential range was approximately +500 to -700mV, the largest of any of the materials studied so far. In the presence of glucose oxidase and glucose in solution the electrode showed a response to added glucose. This response was not however well behaved under the conditions used.

Example 7: Use of Triethylammonium $TCNQ_2$.

Triethylammonium $TCNQ_2$ was drop coated on glassy carbon (area 0.38 $cm^2$).

Of the compounds studied this was the least promising with very large cathodic background currents over nearly the whole "stable" potential range.

Example 8: Use of Quinolinium $TCNQ_2$.

Quinolinium $TCNQ_2$ was packed into a cavity electrode (area 0.03 $cm^2$).

The behaviour of this electrode in the form of a packed cavity electrode was almost identical to that of NMP.TCNQ both in the stable range for the material and in the results obtained in the presence of glucose oxidase and glucose.

Figure 3 shows typical data for detection of glucose using this material as a plot of the current (corrected for background) against concentration of glucose for a Quinolium $TCNQ_2$ packed cavity electrode with Glucose Oxidase. Area 0.03 $cm^2$, E = 50 mV.

A: Where the electrode was covered with a dialysis membrane and a solution of 2.06 mg/ml Glucose Oxidase.

B: Where the electrode was dipped in 2.06 mg/ml Glucose oxidase for 1 hr and then washed before use without a

membrane.

C: Where the same electrode as B was used, but after storage in buffer solution overnight.

Example 9: A Glucose sensor Based on TTF.TCNQ and Glucose Oxidase.

TTF.TCNQ appears to have particular utility as a one dimensional organic conductor, especially with Glucose Oxidase (1.1.3.4) in a glucose sensor. Figure 4 shows an electrode according to the present invention which employs TTF.TCNQ.

9a) Preparation of Electrodes.

In figure 4, the electrode body consists of a platinum wire (2) press-fitted into a Teflon surround (3) so as to leave a cavity (1) approximately 1 mm deep.
TTF.TCNQ was prepared by the method in the literature C.D. Jaeger and A.J. Bard, J.A.C.S. 101, 1690 (1979) as a black crystalline solid.

The TTF.TCNQ was mixed with polyvinyl chloride (Aldrich) in the ratio 0.1 : 1.4 by weight and the mixture was made up into a thick slurry with a little purified tetrahydrofuran. This slurry was then packed into the

cavity of the electrode and the surface smoothed off flush with the Teflon mantle.

The tetrahydrofuran was allowed to evaporate at room temperature and pressure for 30 mins.

Glucose Oxidase (Obtained from Sigma) was then adsorbed onto the surface of the electrode by immersing the packed electrode in a solution of 5 mg of Glucose Oxidase in 1 ml of buffer (150 mM NaCl, phosphate pH 7.4) for 8 hrs at room temperature.

The electrode was then washed with copious amounts of buffer solution before being transferred to an electrochemical cell containing the degassed buffer solution at 37°C. The electrode was potentiostatted at 0 mV against a saturated calomel electrode until the residual current had fallen to less than 2 nA (for a 3 $mm^2$ electrode); this took about 2 hrs.

Once the electrode had been conditioned in this way subsequent stabilisation of the background current took only about 15 mins, or less if the period of disconnection was brief.

9b) Response of the Electrode

The response of the electrode to glucose was studied by adding successive aliquots of 1.0 M glucose in phosphate buffer to the solution in the electrochemical pot. Both the stability of the response to glucose and the stability of the electrode to storage and subsequent use were investigated as described below.

Figure 5 shows typical results for the addition of glucose to the solution over a period of 8 mins. These results are plotted as a function of the added glucose concentration in Figure 6,curve (a). The electrode responds to glucose from less than 0.1 mM to greater than 6mM with a sensitivity of 10μM.

9c) Stability of the Electrode

Two aspects of the stability of the electrode have been investigated. Firstly the stability of the current at constant glucose concentration and secondly the stability of the electrode towards storage and subsequent re-use.

In all cases when the electrode was potentiostatted in a glucose solution the current was found to be stable (for example in 0.6 mM glucose the current remained constant at 169 ± 1 nA in 6.5 hrs.).

When the electrode was left running in glucose overnight the initial current fell linearly from 672 to 480 nA in 17 hrs. However when the same electrode was removed from the test solution washed with buffer and then retested by adding aliquots of glucose to a fresh solution of buffer the response was found to be practically the same as the results obtained under the same regime the day before.

Figure 6 shows a plot of the correct current as a function of the concentration of added glucose for a TTF.TCNQ electrode with adsorbed Glucose Oxidase and no membrane.

a: Initial response.

b: Re-used after running overnight.

C: After storage for 1 week in buffer solution containing glucose.

It can be seen from the above that the observed decrease in the overnight run does not arise from a decay in the activity of the electrode. It is possible that the decay is due to inhibition by product but this conjecture needs to be further investigated.

When the electrode was stored for one week at room temperature in an air-saturated buffer solution of 0.9 mM glucose and then retested it was found that the

electrode still responded to glucose concentration. Results from this experiment are also shown in Figure 6 curve (c). From the Figure we can see that the electrode is very stable to storage without any special precautions. Interestingly, after storing the electrode in buffer containing no glucose for 8 days the response to glucose was reduced in magnitude and was significantly more sluggish (Figure 7) 40 mM glucose in pH 7.4 phosphate buffer, room temperature .

9d) Continuous Operation

Figure 13 shows the results of a further test into the stability of the electrode under conditions of continuous operation. Glucose Oxidase was the enzyme chosen in this case as it was the best characterised of the range of assay systems inventigated.
A 3.5mg/ml solution of glucose oxidase was entrapped on a TTFTCNQ packed cavity electrode using tissue paper and a membrane. The electrode was set up in 20ml of degassed pH 7.4 phosphate buffer, background current was allowed to decay and additions of 1M glucose in phosphate buffer made. The electrode was then left at a constant potential of +50mV in a 30 mM glucose solution for 65 hours. The glucose solution was then replaced by fresh buffer, the system was degassed and additions of 1M glucose were again made. The electrode was then left

at the same potential for a further 100 hours of 40mM glucose solution at +50mV (Method of enzymatic analysis Vol II p.149 Verlag Chemie) and at room temperature. Each day the solution was degassed and the current recorded.

After 65 hours of operation the current/concentration profile showed a slight alteration in slope. Kinetic analysis of this data has suggested that this may be due to deterioration of the membrane.

As a consequence of its low background the electrode described is sensitive to glucose concentration changes of less than 10 μM over a wide concentration range. It operates without a membrane or any additional mediator. The enzyme is irreversibly adsorbed onto the electrode and no special immobilisation techniques are required. The electrode shows excellent stability of response to glucose and upon prolonged storage (1 week) at room temperature in air-saturated buffer containing glucose. Finally when the electrode needs to be regenerated this is readily achieved by polishing the surface and then re-adsorbing glucose oxidase from solution.

<u>Example 10 Use of the electrode with other flavoproteins</u>

In addition to elctrodes which employ Glucose Oxidase, the present invention extends to systems which combine TTFTCNQ with other enzymes. Four other flavoprotein /TTFTCNQ systems will be exemplified.

Packed cavity (4mm diameter) and drop coated glassy carbon electrodes were prepared substantially as described above. These electrodes were used in conjunction with a Pt gauze counter electrode, and a saturated calomel reference electrode in a three electrode system. The working electrodes were held at +50mV with respect to the saturated calomel reference electrode using a potentiostat.

Current was recorded as a function of time using a Bryans 29000 A4 chart recorder at 50s/cm. Packed cavity electrodes were used in a vessel of 25ml total volume; drop coated glassy carbon electrodes were used in a vessel of 2ml total volume. All experiments were carried out at room temperature.
Doubly distilled water was used throughout. Solutions were degassed before use by bubbling $O_2$ free $N_2$ through for 15 minutes The membranes used were dialysis tubing boiled in 1% w/w $Na_2CO_3$ for 10 minutes and stored in Tris (BDH)/EDTA solution.

<u>EXAMPLE 10a) Choline Oxidase</u> (EC 1.1.3.17)

Choline $+O_2$ = betaine aldehyde $+H_2O_2$

Choline chloride and choline oxidase as used in this example were both obtained from Sigma. The choline oxidase used was 15U/mg. It should be noted that there is no prior known electron acceptor, other than $O_2$ for choline oxidase.

A 1mg/ml solution of choline oxidase in pH 7.4 phosphate buffer was entrapped on a TTFTCNQ packed cavity electrode using dialysis membrane. The electrode was set up in 20 ml of degassed pH 7.4 phosphate buffer and background current was allowed to decay (to 10nA in 30 minutes). Choline chloride (0.1M in pH 7.4 phosphate buffer) was then added using a micro-litre syringe. A similar experiment was carried using an electrode which had been dipped in a 1mg/ml choline oxidase solution in an ice bath, for 1 hour in order to absorb enzyme onto the electrode surface.

With the enzyme entrapped by a membrane the electrode responded to additions of choline (Fig. 8), a typical response time being 100 seconds (Km for the free enzyme =1.2 mM). Without the membrane no response was obtained.

<u>Example 10b- Xanthine Oxidase</u> (EC 1.2.3.2)

$$Xanthine + H_2O + O_2 = urate + H_2O_2$$

This enzyme exhibits low specificity and attacks a number of aldehydes, purines, pteridines, pyrimidines, ozapurines and other heterocyclic compounds. Ferricyanide, cytochrome c and several organic dyes can replace $O_2$ as an electron acceptor.

The materials used in this example were; xanthine (sigma grade III 98 - 100%), xanthine oxidase (Sigma grade III from buttermilk, suspension in 3.2 M $(NH_4)_2$ $SO_4$ 10mM sodium phosphate buffer pH 7.8 containing 1 mM EDTA 1.25u/mg, 0.84 25u).

Xanthine oxidase was entrapped on a TTFTCNQ packed cavity electrode using dialysis membrane. The electrode was set up in 15ml of degassed pH 7.4 phosphate buffer and the background current allowed to decay (to 65nA in 1 hour). One ml addition of a 0.55mM xanthine solution in buffer was then made. The experimental procedure given above was also carried out using an electrode onto which an enzyme has been absorbed by dipping the electrode in xanthine oxidase solution for 1 hour in an ice bath. Typical response time was 50 seconds, Km for the free enzyme 3.6 µM.

With a membrane, the electrode responded to additions of xanthine up to 150mM xanthine where the system became saturated (Fig. 9, 10). Without the membrane, responses were observed but the current obtained were too small for a detailed quantitative analysis.

<u>Example 10c- L-amino acid Oxidase</u> (EC 1.4.3.2)

L-a. a. + $H_2O$ + $O_2$ = 2-oxoacid + $NH_3$ + $H_2O_2$.

The enzyme is available from a number of sources, each with a different specificity. The preparation used here was from diamond rattlesnake venom and attacks: Leu, Methionine, Phenylalanine, norvaline, norleucine, cysteine, cyr, cry, histidine, arginine, ornithine and citralene.

The materials used were; L-phenylalanine (Sigma), L-amino acid oxidase (Sigma, crude crotalus adamanteus diamond rattlesnake) venom type 111u, 0.44U/mg dissolved in 1ml pH 6.5 phosphate buffer.

Due to the hazardous nature of the reagent, in order to minimise spillages a different procedure for enzyme entrapment was used. Tissue (Whatmans 105 lens tissue) was placed onto a TTFTCNQ packed cavity electrode and soaked in enzyme. Dialysis membrane was then placed over the tissue and secured onto the electrode with an

O-ring. The electrode was set up in 20ml of degassed pH 6.5 phosphate buffer and the background current allowed to decay (to 6nA in 45 minutes). A 0.1M buffered solution of L-phenylalanine was then added in 20 µl portions. Typical response time was 300 seconds.The electrode was then left overnight in buffer/substrate solution and the experiment repeated. The membrane and tissue paper were then removed and experimental procedure repeated again.

Immediately after preparation the enzyme responded to substrate but current continued to rise for over an hour after an addition. After leaving overnight a stable current was obtained 5 minutes after addition (Fig. 11). This effect is thought to be due to the adsorption of enzyme onto the electrode. Without the membrane responses to substrate were still obtained at the same response times but current were smaller and the electrode became saturated at a lower substrate concentration (Fig. 11).

<u>Example 10d -D-amino acid Oxidase (EC 1.4.33)</u>

D-amino acid + $O_2$ = 2 - oxoacid + $NH_3$ +$H_2O_2$.

The enzyme will attack straight and branch-chain, sulphur-containing and ring-containing amino acids,

$O_2$, can to a limited extent, be replaced by other electron acceptors.

The materials used in this example were; D-alanine (Sigma), D-amino acid oxidase (Sigma from porcine kidney crystalline suspension in pH 3.2 $(NH_4)_2SO_4$ 14U/mg), 5mg made up in 1ml of pH 8.0 0.1M Tris buffer (BDH) containing 0.1M $Li_2SO_4$ giving a 70U/ml solution.

D-amino acid oxidase was entrapped on a TTFTCNQ packed-cavity electrode using dialysis membrane. The electrode was set up in 20ml of degassed pH 8.0 Tris buffer and the background current was allowed to decay (to 35nA in 45 minutes). Microlitre additions of 1M D-alanine in pH 8.0 Tris buffer were then made )typical response time 18 minutes) The run was repeated, firstly with the membrane in place and then with it removed.(typical response time 6 minutes).

Example 11

The electrochemically inert behaviour of the phosphate ion $(H_nPO_4^{n-3})$ in aqueous solution means that techniques for the determination of phosphate are inherently indirect.

The classical determination of phosphate involves the extraction of the ion into acid solution, the reaction of the phosphate with a molybdate salt to form a phosphomolybdate which is reduced to molybdenum blue and measured colourimetrically. Alternatively, the phosphomolybdate may be reduced electrochemically and the concentration of the phosphate calculated from the current resulting from this reduction. Unfortunately, these techniques suffer from interferences from ions such as silicate and arsenate and procedures to exclude these ions must be taken in order to guarantee accuracy.

As an alternative to this inorganic technique, the specificity of various enzymes may be utilised to detect phosphate. Several techniques have recently been proposed but these tend to involve the use of complex enzyme systems which are expensive and somewhat unstable. As an alternative to these systems it has been proposed to use the enzyme pyruvate oxidase which requires phosphate as a substrate in the reaction

$H_3PO_4$ + pyruvate + $O_2$ $H_2O_2$ + $CO_2$ + acteylphosphate (1).

The hydrogen peroxide produced can then be measured in various ways. Firstly, the reaction

$H_2O_2$ + MBTH + DMA indamine (2)

can be used, where MBTH is 3-methyl-2-benzothiazolinonehydrazone and DMA is dimethyl aninline. This reaction produces a dye that can be measured colourimetrically but requires the presence of another enzyme peroxidase.

This system, as set out by Tabata and Murachi can be simplified by use of organic conductors such as $TTF^{+}TNCQ^{-}$ or NMP $^{+}TNCQ^{-}$ which is the system now proposed.

The enzyme is first allowed to adsorb onto an electrode made from one of these conductors e.g. $TTF^{+}TCNQ^{-}$ and membrane placed over the whole electrode, using the system generally as disclosed in previous examples. The electrode acts as a short cut for the enzyme that is reduced during the reaction with the pyruvate and phosphate and requires some means to regain its initial oxidation state. In Tabata and Murachi's system the route chosen is via the reduction of oxygen, in the present system the enzyme is oxidised by the electrode and thus the current required for this oxidation can be monitored and related to the rate at which the enzyme is turning over the substrates. From Figure 14 it can be seen that the rate, or current increases with the concentration of phosphate and thus allows this set-up to be used as a route by which the presence of phosphate

may be determined in aqueous solutions.

## Variants on the Electrodes

Although little direct use of the one-dimensional conduction properties of the material(X) is made in the embodiments described above, it is envisaged possible that further exploitation of this feature may be undertaken. For example, an electrode can be envisaged which comprises a needle-like single crystal of the material(X) with a body containing enzyme on a face thereof and electrical connections made to a further face thereof. Correct selection of the faces will ensure that there is little conduction of charge except through the body containing enzyme.

Various modifications may be made within the scope of the present invention. For example, it will be apparent that while the invention has primary relevance to a sensor electrode, especially such an electrode specific for glucose, it also relates to the combination of such an electrode and temporary or permanent implantation means, e.g. a needle-like probe. Also, such an electrode, connected or connectable, with signal or control equipment,constitutes an aspect of the invention. The electrodes according to the invention permit the manufacture of an improved macro-sensor for

use in hospital analytical glucose sensing instruments. The electrodes of the invention, on the macro-scale could be incorporated into simple, cheap electronic digital read-out instruments for surgery or for home use.

Use of a small version of the macro-sensor would be possible in a device which automatically takes a blood sample from the finger, brings it into contact with the sensor, amplifies the signal and gives a digital readout.

Claims.

1. An electrode for use in assay system which electrode exhibits at an operative surface (a) a material having one-dimensional electrical conduction properties and (b) a flavoprotein enzyme chosen from glucose oxidase, L-aminoacid oxidase, D-aminoacid oxidase, choline oxidase, xanthine oxidase, monoamine oxidase and pyruvate oxidase.

2. An electrode as claimed in claim 1, in which the said material is an organic conductor.

3. An electrode as claimed in claim 1 or 2, in which the said material is a derivative or salt of 7,7,8,8-tetracyano p-quinodimethane TCNQ.

4. An electrode as claimed in claim 3 in which the TCNQ material further comprises copper (dipyridylamine), tetrathiafulvalene, ferrocene, triethylamine or quinoline in the form of positive ions.

5. An electrode as claimed in claim 3 in which the enzyme is choline oxidase and the TCNQ salt is the tetrathiafulvalene salt.

6. An electrode as claimed in claim 3 in which the

enzyme is pyruvate oxidase and the TCNQ salt is the tetrathiafulvalene salt or the N-methyl-phenazine salt.

7. An electrode for use in an assay system which electrode exhibits at an operative surface (a) a material having one-dimensional electrical conduction properties chosen from copper (dipyridylamine), tetrathiafulvalene, ferrocene, triethylamine and quinoline salts of 7,7,8,8-tetracyano p-quinodimethane, (TCNQ) together with (b) an enzyme located at least at the said operative surface of said electrode whereby charge is transferred to said electrode when the enzyme is catalytically active.

8.An electrode as claimed in claim 7 in which the enzyme is a flavoprotein.

9.An electrode as claimed in any one of the preceding claims 1, 2, 3, 4, 7 or 8, in which a second enzyme is also located at least at the said operative surface of the said electrode, in addition to said first enzyme, said second enzyme being active to convert a second-enzyme substrate to a first-enzyme substrate whereby said first enzyme when catalytically active provides an electrical signal related to the concentration of the second-enzyme substrate.

10.An electrode as claimed in claim 1, 2, 3 4, 7 or 8 in which a substrate for a second enzyme is located at least at the said operative surface of the said electrode, said substrate being convertible by said second-enzyme to a first-enzyme substrate whereby said first enzyme when catalytically active provides an electrical signal related to the active concentration of the second enzyme.

11. A method of assay in which there is contacted with a liquid vehicle containing a component to be selectively detected, measured or monitored an electrode as claimed in any one of claims 1 and 8, wherein the enzyme on said electrode is specific to catalyse a reaction of the said component.

12. A method of assay in which there is contacted with a liquid vehicle containing flavoprotein enzyme, chosen from glucose oxidase, L-aminoacid oxidase, D-aminoacid oxidase, choline oxidase, xanthine oxidase, monoamine oxidase and pyruvate oxidase, together with a component selectively catalysed thereby to be detected, measured or monitored, an electrode which exhibits at an operative surface a material having one-dimensional electrical conduction properties.

13. A method as claimed in claim 12 in which the said one-dimensionally conductive material is a salt of TCNQ.

14. A method as claimed in claim 13 in which the salt is $TTF^{+}TCNQ^{-}$.

15. A method of assay in which there is contacted with a liquid vehicle containing a flavoprotein enzyme and a component selectively catalysed thereby to be detected, measured or monitored, an electrode which exhibits at an operative surface a TCNQ salt chosen from the copper (dipyridylamine), tetrathiafulvalene, ferrocene, triethylamine and quinoline salts.

Tetracyanoquinodimethane
(Acceptor)

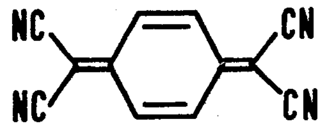

(a)

Copper di-pyridylamine

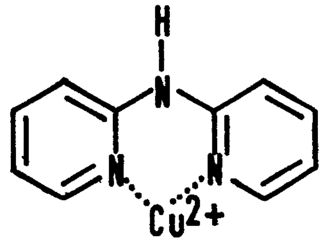

(b)

Tetrathiafulvalene

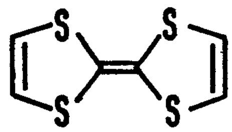

(c)

Ferrocene

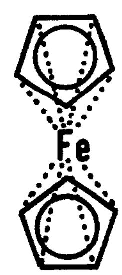

(d)

Triethylammonium

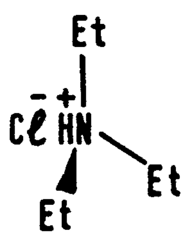

(e)

Quinoline

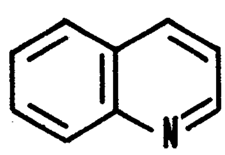

(f)

FIG.1.

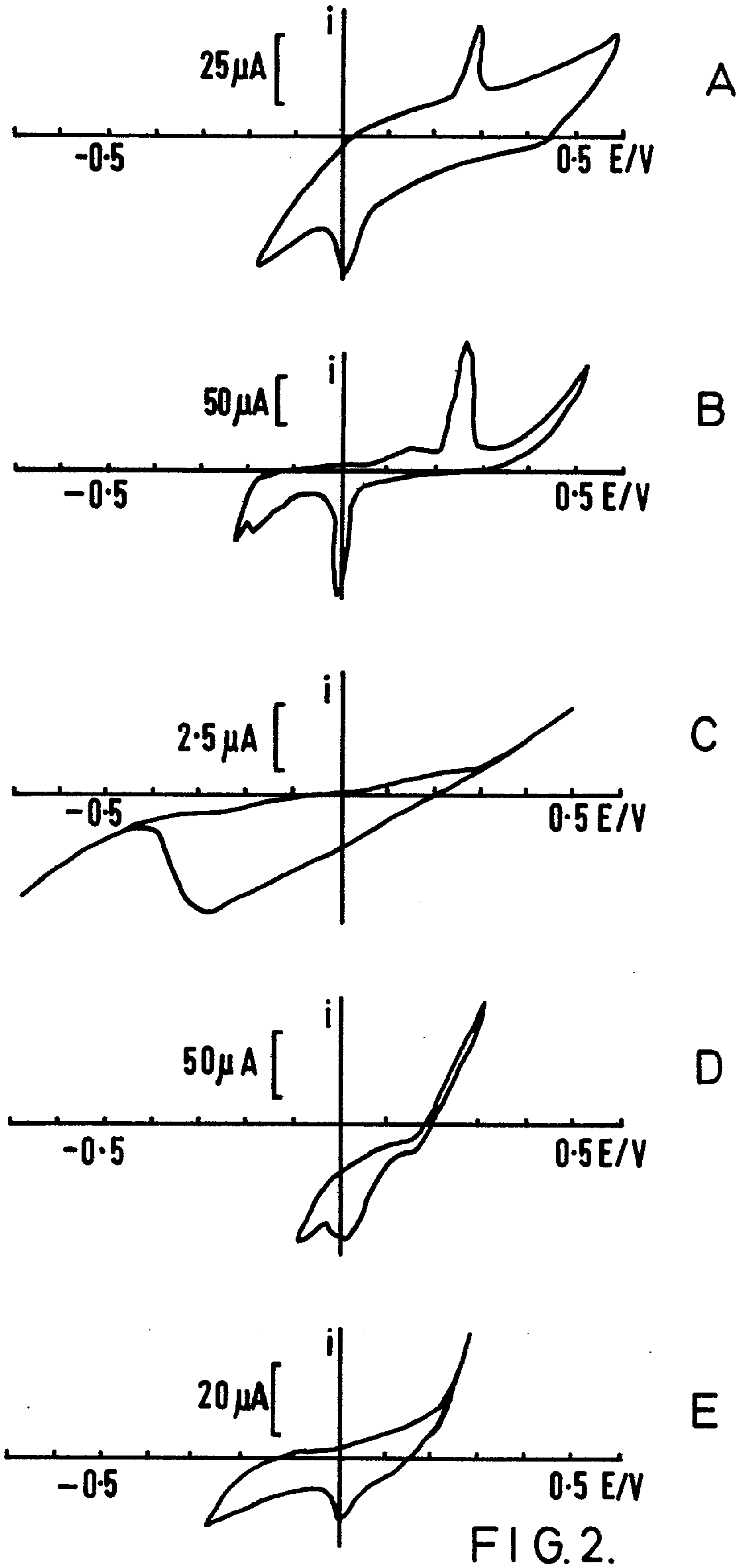
i
25μA
-0·5
0·5 E/V
A
i
50μA
-0·5
0·5 E/V
B
i
2·5μA
-0·5
0·5 E/V
C
i
50μA
-0·5
0·5 E/V
D
i
20μA
-0·5
0·5 E/V
E

FIG.2.

0184909



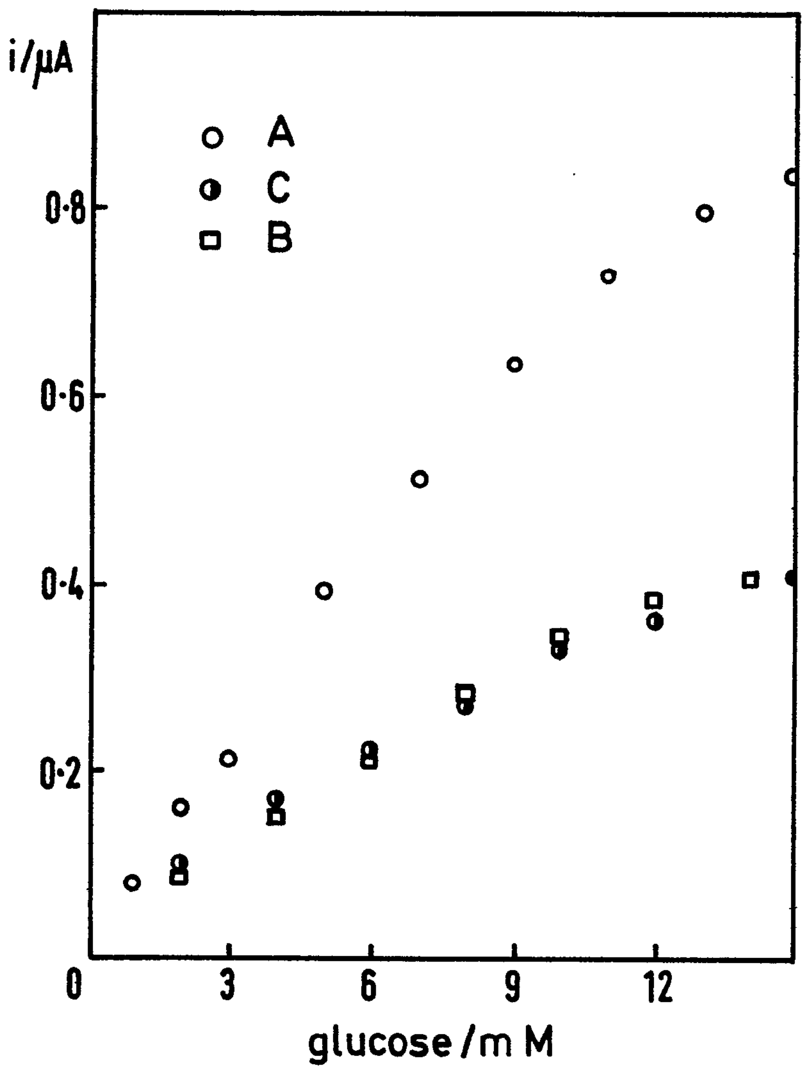


FIG.3.

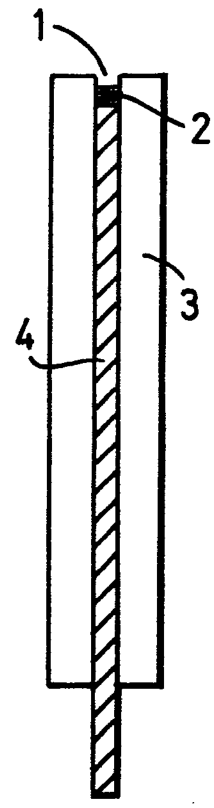


FIG.4.

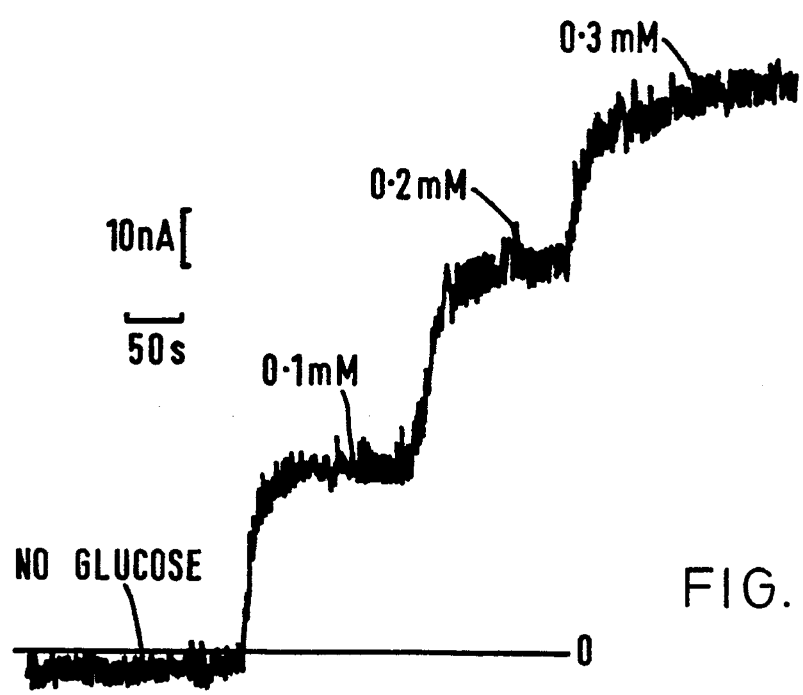


FIG.5.

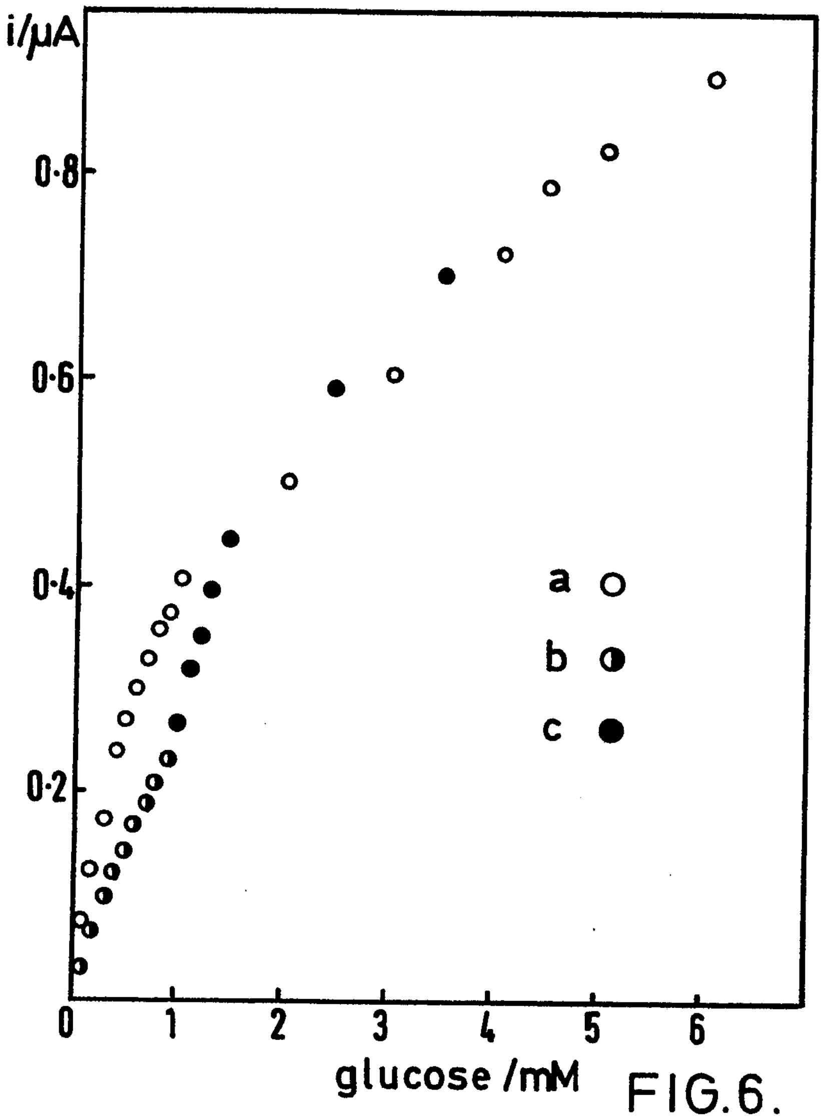

i/μA
0·8
0·6
0·4
0·2
0
1
2
3
4
5
6
glucose /mM
a
b
c


FIG.6.

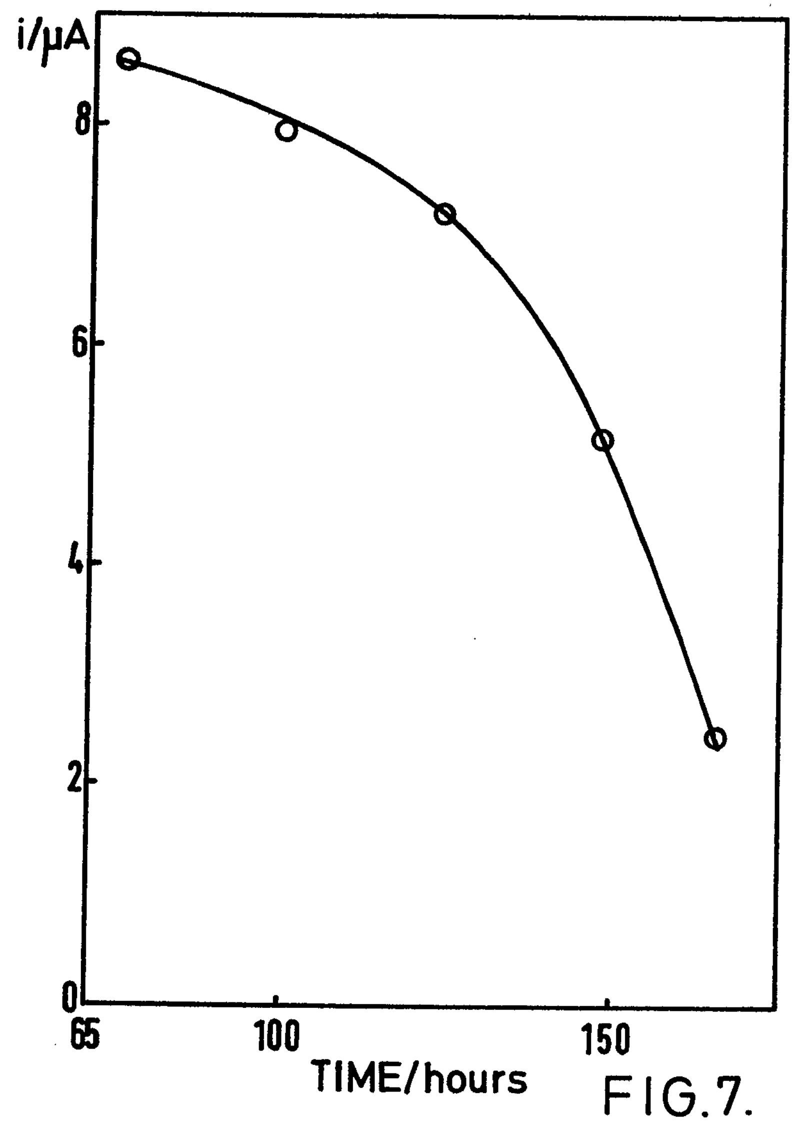

i/μA
8
6
4
2
0
65
100
150
TIME/hours


FIG.7.

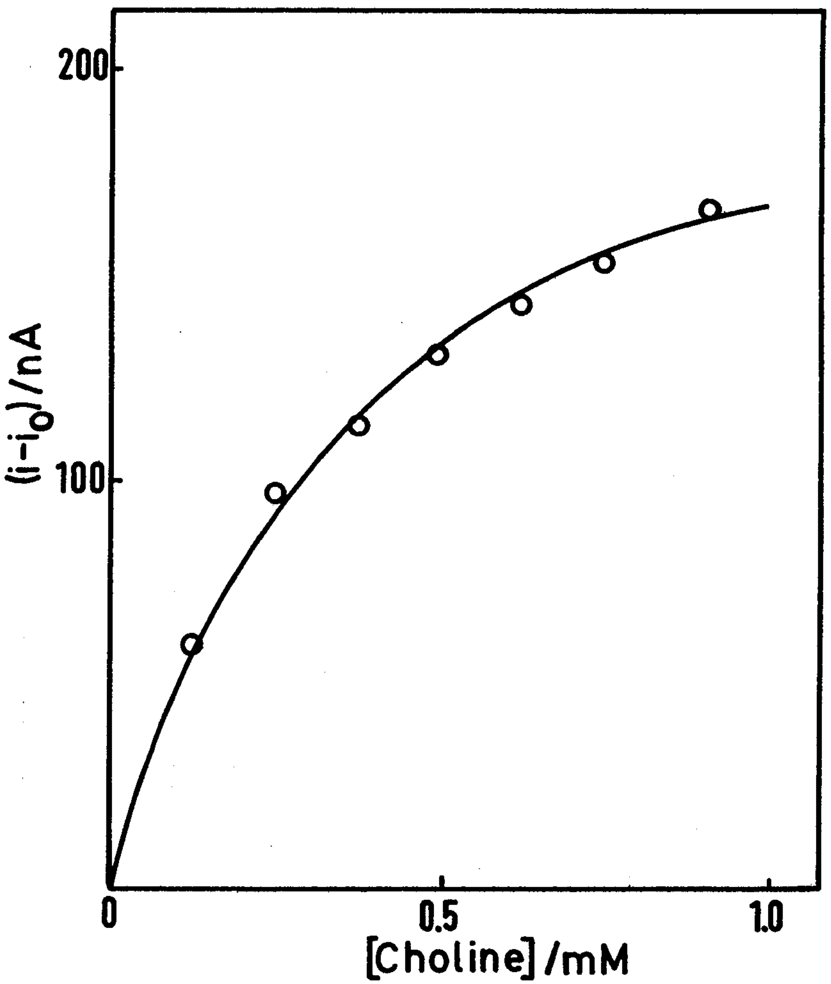
200
100
(i-i₀)/nA
0
0.5
1.0
[Choline]/mM

FIG.8.

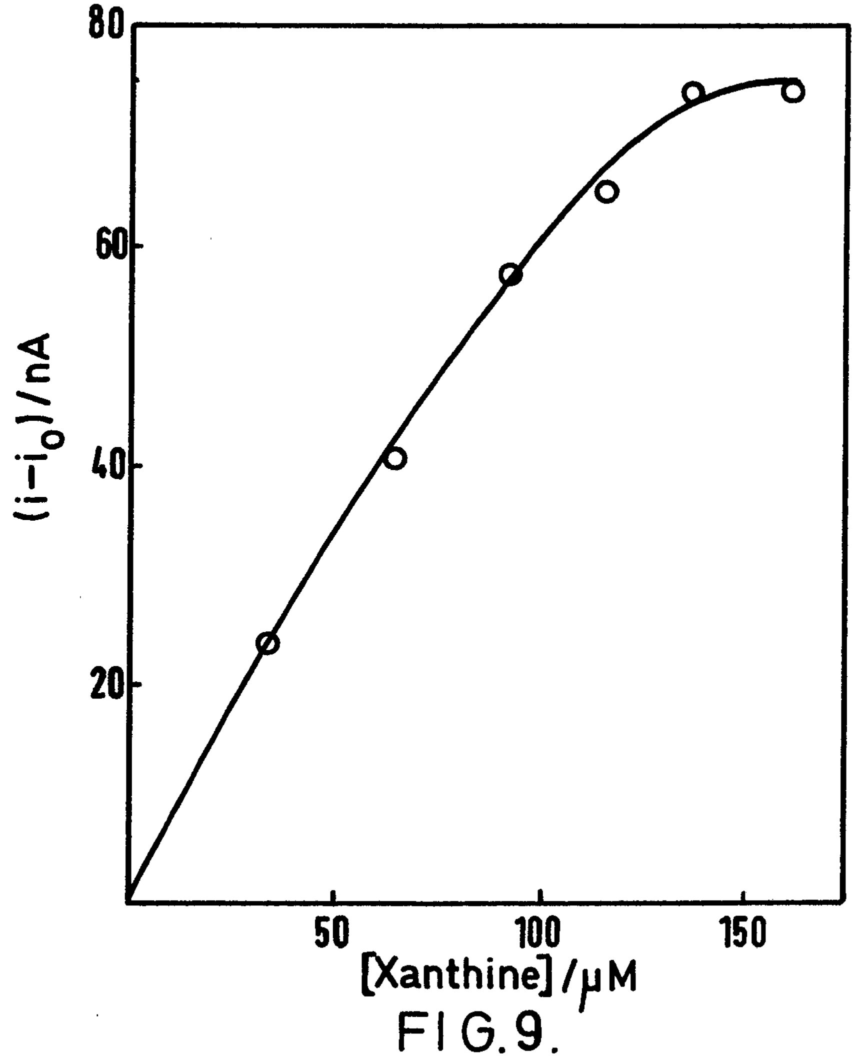
80
60
40
20
(i-i₀)/nA
50
100
150
[Xanthine]/µM

FIG.9.

0184909



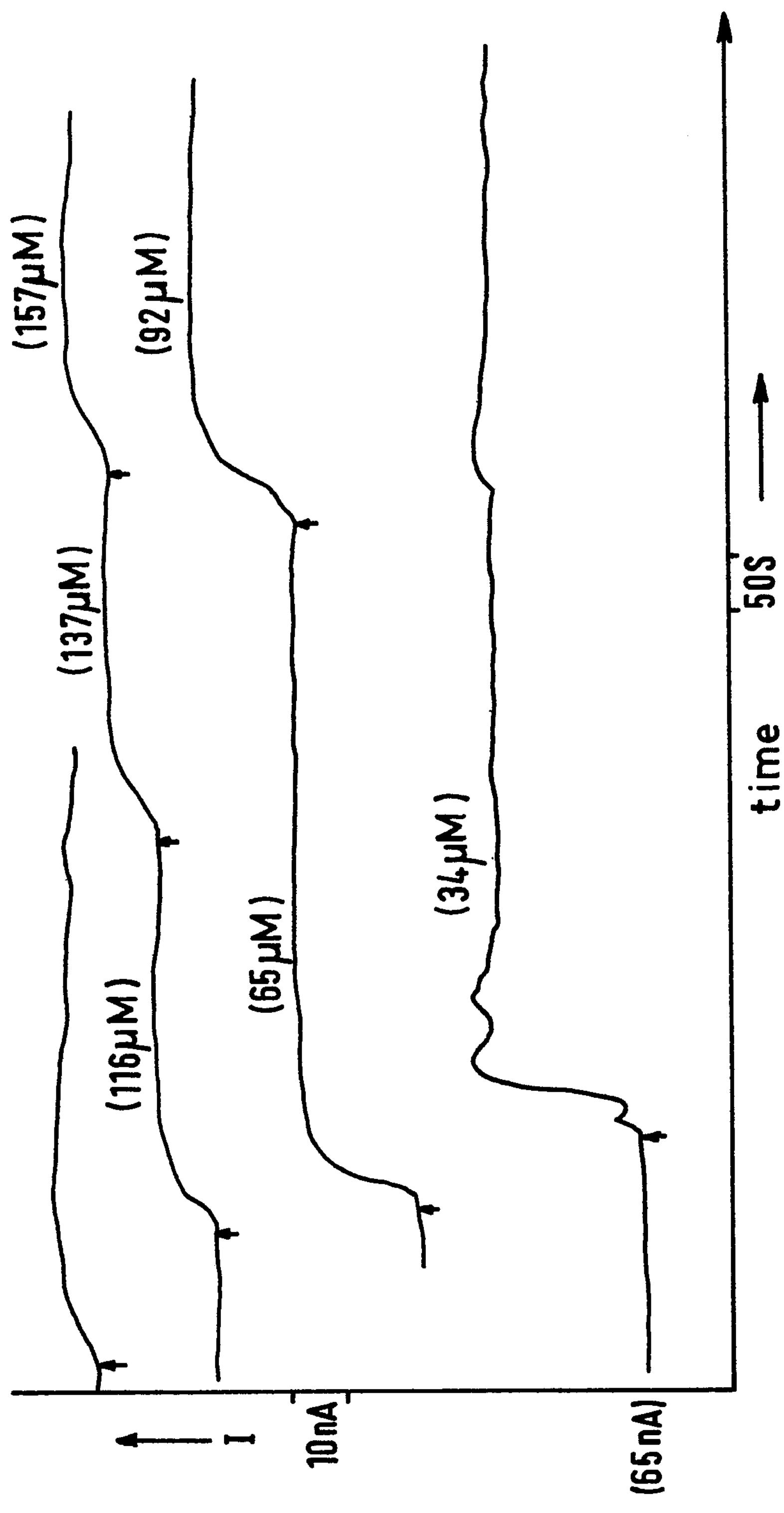


FIG. 10.

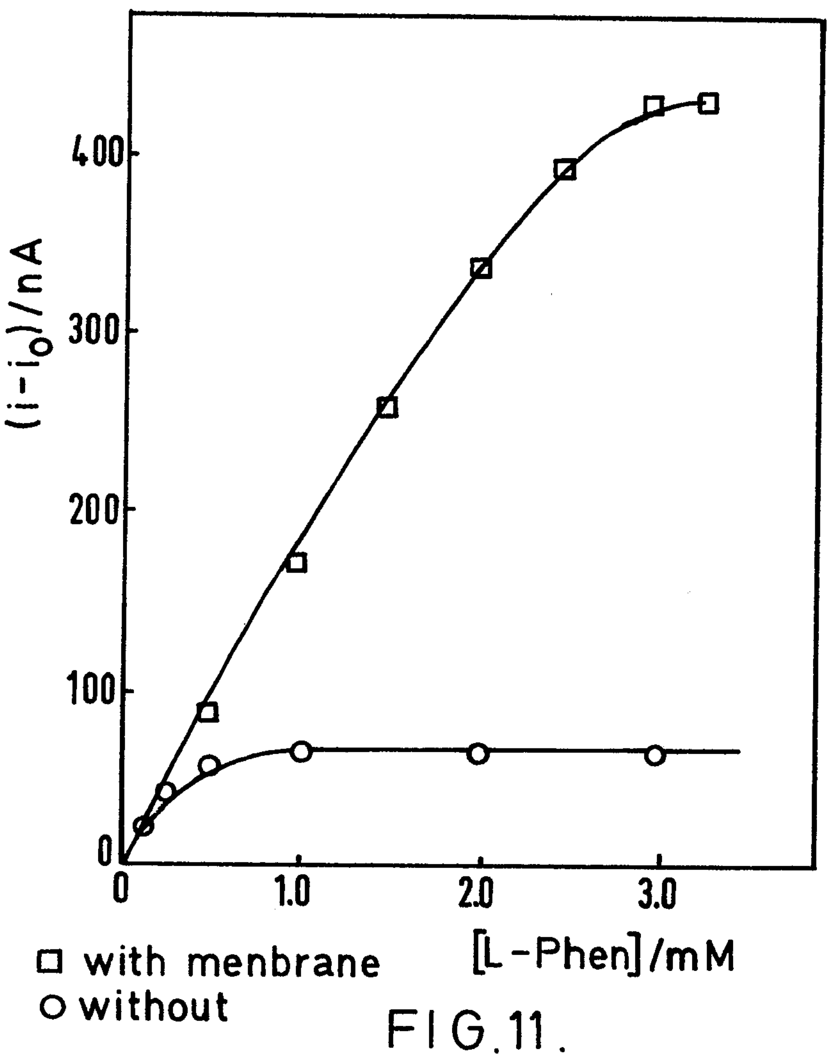
(i-io)/nA
400
300
200
100
0
0
1.0
2.0
3.0
[L-Phen]/mM
□ with menbrane
○ without

FIG.11.

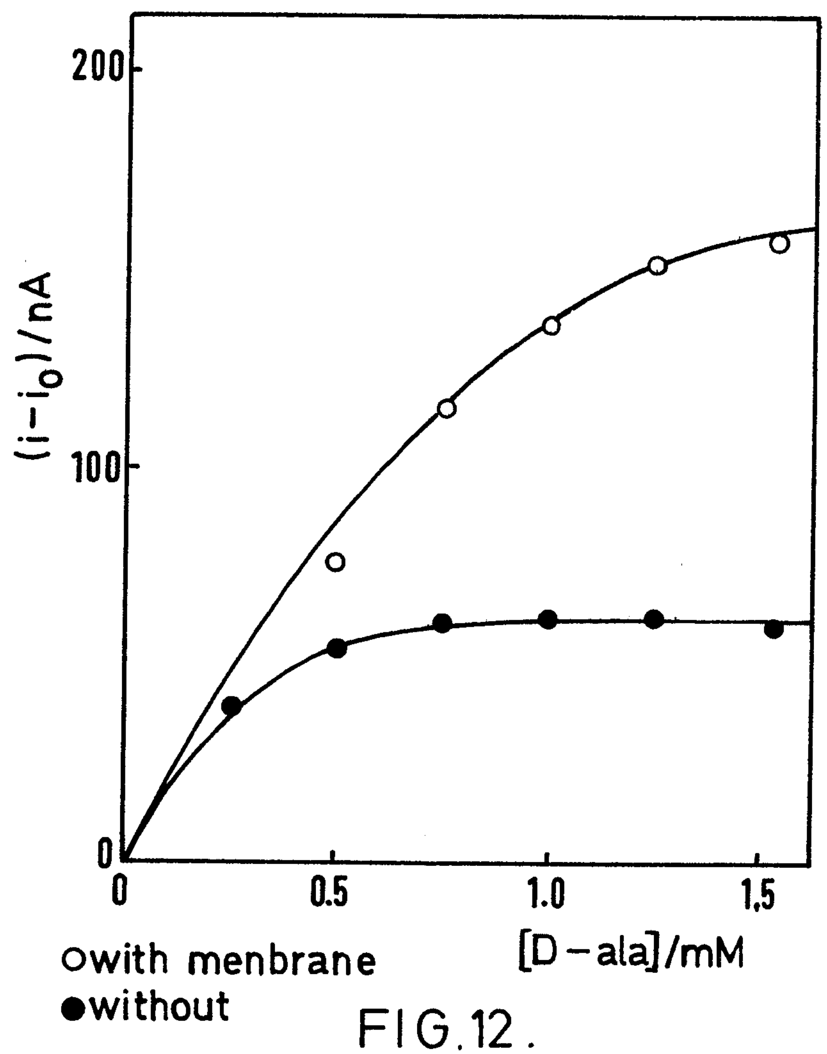
(i-io)/nA
200
100
0
0
0.5
1.0
1,5
[D-ala]/mM
○with menbrane
●without

FIG.12.

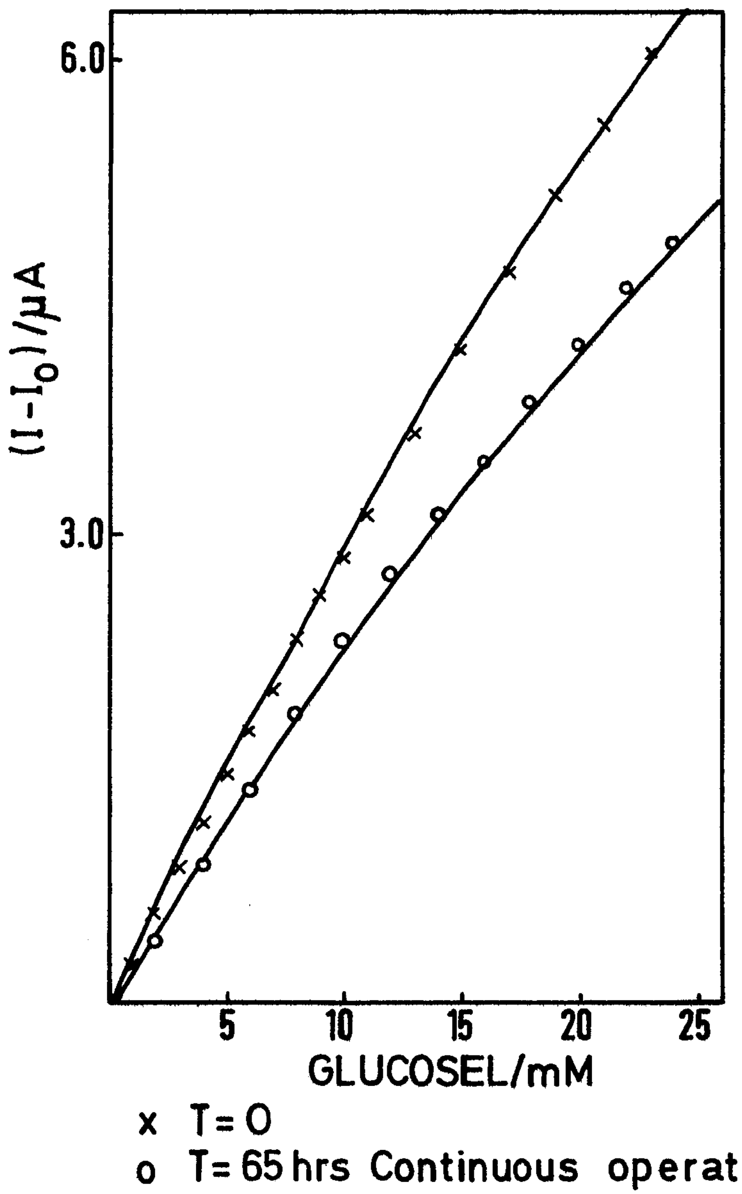
(I-I$_o$)/μA
6.0
3.0
5
10
15
20
25
GLUCOSEL/mM
x T=O
o T=65 hrs Continuous operation

FIG.13.

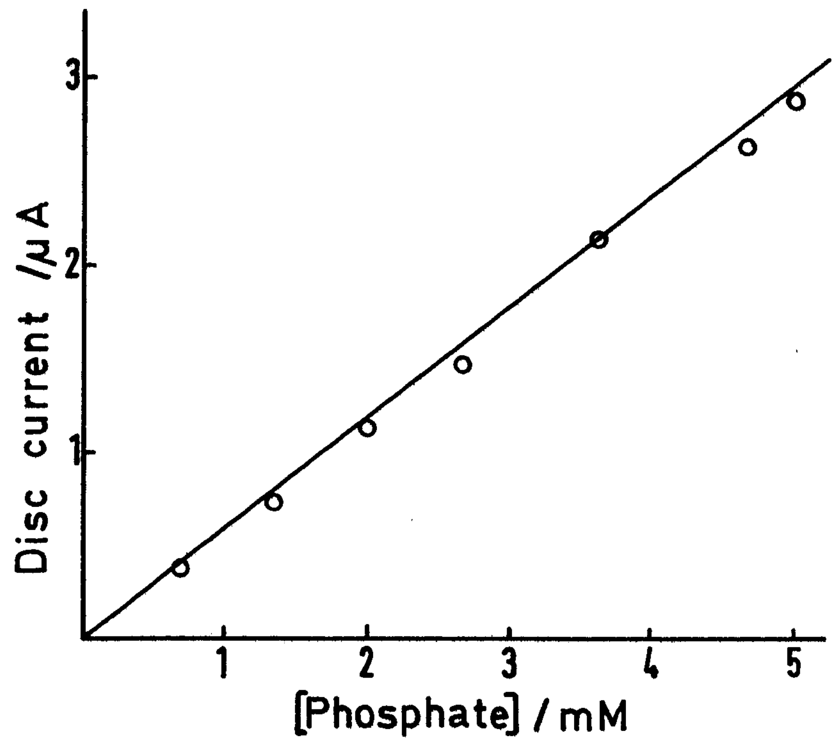
Disc current /μA
1
2
3
4
5
[Phosphate] / mM

FIG. 14.